# EUROPÄISCHE PATENTSCHRIFT

(11) **EP 0 593 873 B1**
(45) Veröffentlichungstag und Bekanntmachung des Hinweises auf die Patenterteilung: **06.09.1995**
(21) Anmeldenummer: 93113389.6
(22) Anmeldetag: 21.08.1993
(51) Int. Cl.: C07C 15/073, C07C 15/46, C07C 7/04

(54) **Verfahren zur Trennung von Ethylbenzol/Styrol durch Destillation eines flüssigen, überwiegend aus Styrol und Ethylbenzol bestehenden Gemisches**
Process for the separation of ethylbenzene/styrene by distillation of a liquid mixture consisting mainly of styrene and ethylbenzene
Procédé pour la séparation d'éthylbenzène/styrène par distillation d'un mélange liquide consistant essentiellement en styrène et éthylbenzène

(30) Priorität: 21.10.1992 DE 4235431
(43) Veröffentlichungstag der Anmeldung: 27.04.1994
(73) Patentinhaber: HÜLS AKTIENGESELLSCHAFT, 45764 Marl (DE)
(72) Erfinder: Keil, Thomas, Dr., D-46236 Bottrop (DE); Hüwels, Wilhelm, D-45133 Essen (DE); Doll, Georg, D-45131 Essen (DE)

(56) Entgegenhaltungen:
- EP-A- 0 160 553
- US-A- 3 256 355
- US-A- 4 395 310
- Separation Process Plants Bulletin VT 1673/74 May 1982 (Sulzer- Winterthur) Seite 3, 12.4.1

## Beschreibung

Die Erfindung betrifft ein Verfahren entsprechend dem Oberbegriff des Patentanspruchs 1 in Verbindung mit der Zeichnung. Ein solches Verfahren ist aus dem Separation Process Plants Bulletin VT 1673/74, May 1982, Seite 3, 12.4.1, Separation of Ethylbenzene/Styrene, der Fa. Sulzer bekannt. Es ist nicht erkennbar, wie die Destillationskolonne (Rohstyrolkolonne) geregelt wird.

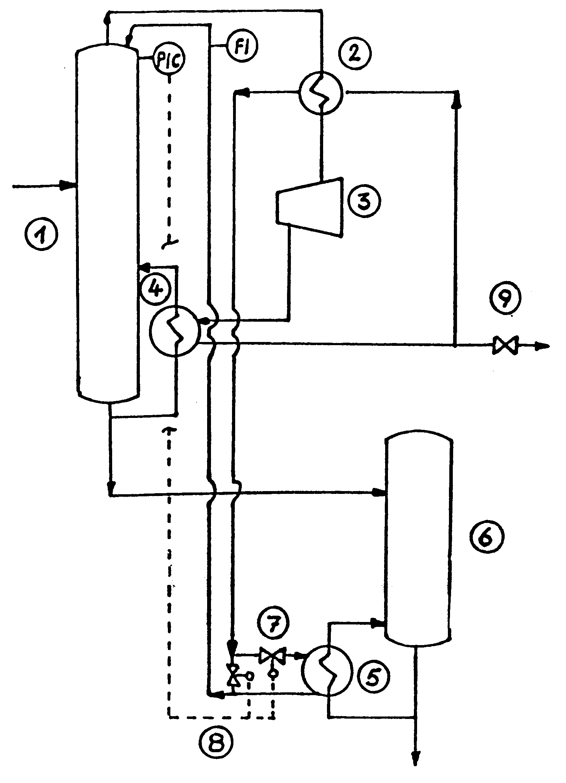

Aus der EP-PS 0 160 553 ist eine Weiterentwicklung des Verfahrens der Fa. Sulzer bekannt. Dabei wird der vom Kopf der Destillationskolonne (2) (Fig. 1) abgezogene Dampf der Niedrigsieder in zwei Teilströme geteilt: Ein erster Teilstrom wird in bekannter Weise durch einen "Vorheizer" (16), in dem der Teilstrom überhitzt wird, zu einem Kompressor (7) zur adiabatischen Kompression geleitet. Ein zweiter Teilstrom wird zu einem Kondensator (5) geleitet (Patentanspruch 1, Merkmal c). Offenbar geht die Kondensationswärme des zweiten Teilstroms dem System im Kondensator (5) verloren. Die Kolonne (2) wird auf komplizierte Weise geregelt. Dabei ist die Destillation sehr stabil.

Die Aufgabe der Erfindung besteht darin, ein Verfahren zur Trennung von Ethylbenzol/Styrol durch Destillation eines flüssigen, überwiegend aus Styrol und Ethylbenzol bestehenden Gemisches bereitzustellen, das die aufgewendete Energie besser nutzt als in den Verfahren des geschilderten Standes der Technik und bei dem die Destillationskolonne (Rohstyrolkolonne) auf möglichst einfache Weise geregelt wird. Dabei soll die Destillation sehr stabil sein.

Die Aufgabe wurde wie in den Patentansprüchen angegeben gelöst.

Das Verfahren der Erfindung umfaßt somit die folgenden Schritte:
- Zuführen eines flüssigen, überwiegend aus Styrol und Ethylbenzol bestehenden Gemisches in ein Mittelteil einer Destillationskolonne (1) vom Typ einer gepackten Säule, die unter vermindertem Druck betrieben wird (Rohstyrolkolonne);
- Abziehen des Dampfes von Komponenten mit niedrigem Siedepunkt (Niedrigsieder) mit Ethylbenzol als Hauptkomponente vom Kopf der Kolonne (1) und Abziehen einer Flüssigkeit von Komponenten mit hohem Siedepunkt (Hochsieder) mit Styrol als Hauptkomponente vom unteren Teil der Kolonne (1);
- Leiten des Dampfes der Niedrigsieder durch einen Überhitzer (2) zu einem Kompressor (3) zur adiabatischen Kompression (Brüdenkompression);
- Einleiten von komprimiertem Dampf aus dem Kompressor (3) in einen Aufkocher (4) der Kolonne (1);
- Rückführen eines Teils der im Aufkocher (4) kondensierten Flüssigkeit aus Niedrigsiedern durch den Überhitzer (2) zum oberen Teil der Kolonne (1) und Abziehen des übrigen Teils der kondensierten Flüssigkeit aus Niedrigsiedern von dem Destillationssystem, wobei der aus dem Überhitzer (2) austretende Teil der kondensierten Flüssigkeit aus Niedrigsiedern in zwei Teilströme geteilt wird:
Ein erster Teilstrom wird durch einen Aufkocher (5) einer Destillationskolonne (6) vom Typ einer (gepackten) Säule, die unter vermindertem Druck betrieben und die mit der Flüssigkeit aus Hochsiedern vom unteren Teil der Kolonne (1) beschickt wird, geleitet (Reinstyrolkolonne) und zum oberen Teil der Kolonne (1) rückgeführt. Somit dient der erste Teilstrom als eine Wärmequelle für die Reinstyrolkolonne (6). In dieser wird das Styrol von höher siedenden Komponenten durch Destillieren über Kopf abgetrennt. Der erste Teilstrom kann alternativ zu einer anderen nutzbaren Wärmesenke geeigneter Temperatur geleitet und dann zum oberen Teil der Kolonne (1) rückgeführt werden.

Ein zweiter Teilstrom wird direkt zum oberen Teil der Kolonne (1) rückgeführt.

In der Leitung des ersten Teilstroms befindet sich ein Ventil (7) und in der Leitung des zweiten Teilstroms ein Ventil (8). Die beiden Ventile stehen mit einer am Kopf der Kolonne (1) vorhandenen Meßstelle für den Druck (PIC) in einem Regelkreis. Mit diesem wird die Kolonne (1) geregelt. Durch diese Regelung wird überraschenderweise ein besonders stabiler Zustand der Kolonne (1) erreicht (Patentanspruch 1).

Das flüssige, überwiegend aus Styrol und Ethylbenzol bestehenden Gemisch kann das Rohprodukt aus der Dehydrierung von Ethylbenzol sein. Es enthält dann als weitere Niedrigsieder Toluol und Benzol sowie Inerte wie Stickstoff und Wasser.

Das Rohprodukt aus der Dehydrierung von Ethylbenzol kann zuvor einer Destillationskolonne zugeführt werden, von welcher Toluol, Benzol, Stickstoff und Wasser über Kopf abgezogen werden. Vom unteren Teil der Kolonne wird dann ein Gemisch abgezogen, das im wesentlichen aus Styrol und Ethylbenzol besteht. Dieses Gemisch wird dann einem Mittelteil der Destillationskolonne (1) zugeführt.

In der Leitung für die beiden vereinigten Teilströme, d. h. in der Rücklaufleitung, befindet sich vorzugsweise eine Meßstelle für die Messung der Rücklaufmenge (FI). Der Kompressor (3) weist vorzugsweise eine Vorrichtung zur Einstellung der Förderleistung, beispielsweise eine Dralldrossel, auf. Die Rücklaufmenge wird durch die Förderleistung eingestellt (Patentanspruch 2).

In einem Mittelteil der Kolonne (1) befinden sich vorzugsweise Meßstellen für die Temperatur. In der Leitung zum Abziehen des übrigen Teils der kondensierten Flüssigkeit aus Niedrigsiedern von dem Destillationssystem befindet sich vorzugsweise ein Ventil (9). Die Temperaturmessung und das Ventil (9) stehen in einem Regelkreis. Mit diesem wird die Kolonne (1) geregelt. Mit dieser Regelung wird erreicht, daß die das Destillationssystem der Kolonne (1) verlassenden Produktströme, d. h. der flüssige Produktstrom aus Niedrigsiedern und der flüssige Produktstrom aus Hochsiedern, deren Zusammensetzung fortlaufend gaschromatografisch ermittelt wird, spezifikationsgerecht sind (Patentanspruch 3).

Die aus dem Aufkocher (4) austretende, kondensierte Flüssigkeit aus Niedrigsiedern durchläuft zweckmäßigerweise eine Destillatvorlage.

Die Zusammensetzung des einem Mittelteil der Kolonne (1) zugefuhrten Gemisches wird zweckmäßigerweise fortlaufend gaschromatografisch ermittelt.

## Patentansprüche

1. Verfahren zur Trennung von Ethylbenzol/Styrol durch Destillation eines flüssigen, überwiegend aus Styrol und Ethylbenzol bestehenden Gemisches, wobei das Verfahren die folgenden Schritte umfaßt:
a) Zuführen des flüssigen Gemisches in ein Mittelteil einer Destillationskolonne (1) vom Typ einer gepackten Säule, die unter vermindertem Druck betrieben wird;
b) Abziehen des Dampfes von Komponenten mit niedrigem Siedepunkt (Niedrigsieder) mit Ethylbenzol als Hauptkomponente vom Kopf der Kolonne (1) und Abziehen einer Flüssigkeit von Komponenten mit hohem Siedepunkt (Hochsieder) mit Styrol als Hauptkomponente vom unteren Teil der Kolonne (1);
c) Leiten des Dampfes der Niedrigsieder durch einen Überhitzer (2) zu einem Kompressor (3) zur adiabatischen Kompression;
d) Einleiten von komprimiertem Dampf aus dem Kompressor (3) in einen Aufkocher (4) der Kolonne (1);
e) Rückführen eines Teils der im Aufkocher (4) kondensierten Flüssigkeit aus Niedrigsiedern durch den Überhitzer (2) zum oberen Teil der Kolonne (1) und Abziehen des übrigen Teils der kondensierten Flüssigkeit aus Niedrigsiedern von dem Destillationssystem,
dadurch gekennzeichnet,
daß der aus dem Überhitzer (2) austretende Teil der kondensierten Flüssigkeit aus Niedrigsiedern in zwei Teilströme geteilt wird, wobei ein erster Teilstrom durch einen Aufkocher (5) einer Destillationskolonne (6) vom Typ einer (gepackten) Säule, die unter vermindertem Druck betrieben und die mit der Flüssigkeit aus Hochsiedern vom unteren Teil der Kolonne (1) beschickt wird, oder zu einer anderen nutzbaren Wärmesenke geeigneter Temperatur geleitet und zum oberen Teil der Kolonne (1) rückgeführt wird und ein zweiter Teilstrom direkt zum oberen Teil der Kolonne (1) rückgeführt wird, wobei sich in der Leitung des ersten Teilstroms ein Ventil (7) und in der Leitung des zweiten Teilstroms ein Ventil (8) befinden, welche mit einer am Kopf der Kolonne (1) befindlichen Meßstelle für den Druck in einem Regelkreis stehen, und so die Kolonne (1) geregelt wird.

2. Verfahren nach Anspruch 1,
dadurch gekennzeichnet,
daß sich in einer Leitung für die beiden vereinigten Teilströme in der Rücklaufleitung eine Meßstelle für die Messung der Rücklaufmenge befindet und der Kompressor (3) eine Vorrichtung zur Einstellung der Förderleistung aufweist, wobei die Rücklaufmenge durch die Förderleistung eingestellt wird.

3. Verfahren nach Anspruch 1 oder 2,
dadurch gekennzeichnet,
daß sich in einem Mittelteil der Kolonne (1) Meßstellen für die Temperatur und in der Leitung zum Abziehen des übrigen Teils der kondensierten Flüssigkeit aus Niedrigsiedern von dem Destillationssystem ein Ventil (9) befinden, wobei die Temperaturmessung und das Ventil in einem Regelkreis stehen und so die Kolonne (1) geregelt wird.

4. Verfahren nach einem der Ansprüche 1 bis 3,
dadurch gekennzeichnet,
daß nichtkondensierte Niedrigsieder und Inerte vom Aufkocher (4) in einen Kondensator geleitet werden, der mit der Vakuumanlage verbunden ist.

## Claims

1. A process for separating ethylbenzene/styrene by distillation of a liquid mixture predominantly comprising styrene and ethylbenzene, the process comprising the following steps:
a) feeding the liquid mixture into a central part of a distillation column (1) of the packed column type which is operated at reduced pressure;
b) withdrawing the vapour of components of low boiling points (low boilers) with ethylbenzene as a major component from the head of the column (1) and withdrawing a liquid of components of high boiling points (high boilers) with styrene as a major component from the bottom part of the column (1);
c) passing the vapour of the low boilers through a superheater (2) to a compressor (3) for adiabatic compression;
d) introducing compressed vapour from the compressor (3) into a reboiler (4) of the column (1);
e) recycling a part of the liquid condensed in the reboiler (4) composed of low boilers through the superheater (2) to the upper part of the column (1) and withdrawing the remaining part of the condensed liquid composed of low boilers from the distillation system,
characterized in that the part of the condensed liquid composed of low boilers leaving the superheater (2) is divided into two part-streams, a first part-stream being passed through a reboiler (5) of a distillation column (6) of the (packed) column type, which is operated at reduced pressure and is fed with the liquid composed of high boilers from the bottom part of the column (1), or being passed to another utilisable heat sink of appropriate temperature and recycled to the upper part of the column (1) and a second part-stream being recycled directly to the upper part of the column (1), a valve (7) being situated in the line of the first part-stream and a valve (8) being situated in the line of the second part-stream, which valves, with a pressure measurement point situated at the head of the column (1), are in a control circuit, and the column (1) being controlled in this manner.

2. A process according to claim 1, characterized in that a measurement point for the measurement of the reflux rate is situated in a line for the two combined part-streams in the reflux line and the compressor (3) is equipped with an apparatus for setting the output, the reflux rate being set by the output.

3. A process according to claim 1 or 2, characterized in that measurement points for the temperature are situated in a central part of the column (1) and a valve (9) is situated in the line for withdrawing the remaining part of the condensed liquid composed of low boilers from the distillation system, the temperature measurement and the valve being in a control circuit and the column (1) being controlled in this manner.

4. A process according to any one of claims 1 to 3, characterized in that the non-condensed low boilers and inert substances from the reboiler (4) are passed into a condenser which is connected to the vacuum unit.

## Revendications

1. Procédé pour séparer l'éthylbenzène et le styrène par distillation d'un mélange liquide constitué essentiellement de styrène et d'éthylbenzène, le procédé comprenant les étapes suivantes :
a) amenée du mélange liquide dans une partie centrale d'une colonne de distillation (1), du type colonne garnie, exploitée sous pression réduite ;
b) soutirage de la vapeur des constituant à bas point d'ébullition dont le constituant principal est l'éthylbenzène, en tête de la colonne (1), et, à la partie inférieure de la colonne (1), soutirage d'un liquide composé de constituants à haut point d'ébullition, dont le constituant principal est le styrène ;
c) envoi de la vapeur des constituants à bas point d'ébullition vers un compresseur (3), par l'intermédiaire d'un surchauffeur (2), pour compression adiabatique ;
d) introduction, dans un rebouilleur (4) de la colonne (1), de la vapeur comprimée provenant du compresseur (3) ;
e) renvoi d'une partie du liquide condensé dans le rebouilleur (4), formé de constituants à bas point d'ébullition, et par l'intermédiaire du surchauffeur (2), vers la partie supérieure de la colonne (1), et soutirage, à partir du système de distillation, du reste du liquide condensé formé de constituants à bas point d'ébullition,
caractérisé en ce que la partie, sortant du surchauffeur (2), du liquide condensé formé de constituants à bas point d'ébullition, est subdivisée en deux courants partiels, un premier courant partiel est envoyé dans un rebouilleur (5) d'une colonne de distillation (6) du type colonne (garnie), qui est exploitée sous pression réduite et qui reçoit le liquide formé de constituants à haut point d'ébullition provenant de la partie inférieure de la colonne (1), ou vers un autre puits thermique utilisable, à température appropriée, et est renvoyée à la partie supérieure de la colonne (1), et un deuxième courant partiel est directement envoyé à la partie supérieure de la colonne (1), une vanne (7) se trouvant dans la conduite du premier courant partiel et une vanne (8) se trouvant dans la conduite du deuxième courant partiel, ces vannes se trouvant dans un circuit de régulation, avec un point de mesure de la pression se trouvant en tête de la colonne (1), ce qui assure la régulation de la colonne (1).

2. Procédé selon la revendication 1,
caractérisé en ce qu'un point de mesure destiné à mesurer le débit de retour se trouve dans une conduite destinée aux deux courants partiels unifiés dans la conduite de retour, et le compresseur (3) comporte un dispositif d'ajustement du débit de refoulement, le débit de retour étant ajusté par le débit de refoulement.

3. Procédé selon la revendication 1 ou 2,
caractérisé en ce que des points de mesure destinés à la température se trouvent dans une partie centrale de la colonne (1), et qu'une vanne (9) se trouve dans la conduite par laquelle le reste du liquide condensé, formé de constituants à bas point d'ébullition, est soutiré du système de distillation, la mesure de la température et la vanne se trouvant dans un circuit de régulation, ce qui assure la régulation de la colonne (1).

4. Procédé selon l'une des revendications 1 à 3,
caractérisé en ce que les constituants à bas point d'ébullition et les matières inertes non condensés provenant du rebouilleur (4) sont envoyés dans un condenseur qui est relié à une installation de vide.
